(19) Europäisches Patentamt
European Patent Office
Office européen des brevets

(11) **EP 3 389 483 B1**

(12) **EUROPÄISCHE PATENTSCHRIFT**

(45) Veröffentlichungstag und Bekanntmachung des Hinweises auf die Patenterteilung:
**03.09.2025 Patentblatt 2025/36**

(21) Anmeldenummer: **16843286.2**

(22) Anmeldetag: **13.12.2016**

(51) Internationale Patentklassifikation (IPC):
*A61B 5/0205* (2006.01)   *A61B 5/024* (2006.01)
*G09B 7/02* (2006.01)   *A61B 5/021* (2006.01)
*A63B 24/00* (2006.01)   *A61M 21/00* (2006.01)
*A61B 5/00* (2006.01)   *A61B 5/16* (2006.01)
*A61N 1/04* (2006.01)   *A61N 1/36* (2006.01)
*A63B 22/00* (2006.01)   *A63B 22/02* (2006.01)
*A63B 71/06* (2006.01)   *G09B 19/00* (2006.01)
*G09B 23/28* (2006.01)   *G16H 20/30* (2018.01)
*A61B 5/374* (2021.01)   *A63B 22/06* (2006.01)

(52) Gemeinsame Patentklassifikation (CPC):
(C-Sets verfügbar)
A61B 5/4836; A61B 5/0205; A61B 5/02438;
A61B 5/16; A61B 5/163; A61B 5/374; A61M 21/00;
A61N 1/36; A61N 1/36003; A63B 22/02;
G09B 7/02; G09B 19/00; G09B 23/28; G16H 20/30;
A61B 5/0022;                           (Forts.)

(86) Internationale Anmeldenummer:
**PCT/DE2016/100580**

(87) Internationale Veröffentlichungsnummer:
**WO 2017/101907 (22.06.2017 Gazette 2017/25)**

(54) **VORRICHTUNG ZUR NEURO-VASKULÄREN STIMULATION**

DEVICE FOR NEUROVASCULAR STIMULATION

APPAREIL DE STIMULATION NEURO-VASCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2015 DE 102015121763**

(43) Veröffentlichungstag der Anmeldung:
**24.10.2018 Patentblatt 2018/43**

(73) Patentinhaber: **neotiv GmbH**
**39104 Magdeburg (DE)**

(72) Erfinder:
• **BERRON, David**
**39104 Magdeburg (DE)**
• **DÜZEL, Emrah**
**14467 Potsdam (DE)**
• **REHSE, Chris**
**10405 Berlin (DE)**
• **HAUPENTHAL, Julian**
**79102 Freiburg (DE)**

(74) Vertreter: **Kröncke, Rolf et al**
**Meissner Bolte**
**Patentanwälte Rechtsanwälte**
**Partnerschaft mbB**
**Plathnerstraße 3A**
**30175 Hannover (DE)**

(56) Entgegenhaltungen:
WO-A1-2015/027079     US-A1- 2015 066 104

• **KRISTINA SCHAAFF: "ENHANCING MOBILE WORKING MEMORY TRAINING BY USING AFFECTIVE FEEDBACK", INTERNATIONAL CONFERENCE ON MOBILE LEARNING, 1 March 2013 (2013-03-01), Lisbon, Portugal, pages 269 - 273, XP055375589, ISBN: 978-972-893-981-6**

• TAKAHASHI M ET AL: "Experimental study toward mutual adaptive interface", ROBOT AND HUMAN COMMUNICATION, 1994. RO-MAN '94 NAGOYA, PROCEEDINGS., 3RD IEEE INTERNATIONAL WORKSHOP ON NAGOYA, JAPAN 18-20 JULY 1, NEW YORK, NY, USA,IEEE, 18 July 1994 (1994-07-18), pages 271 - 276, XP010125417, ISBN: 978-0-7803-2002-4, DOI: 10.1109/ROMAN.1994.365918
• NATHAN THEILL ET AL: "Effects of simultaneously performed cognitive and physical training in older adults", BMC NEUROSCIENCE, BIOMED CENTRAL, LONDON, GB, vol. 14, no. 1, 23 September 2013 (2013-09-23), pages 103, XP021158634, ISSN: 1471-2202, DOI: 10.1186/1471-2202-14-103

(52) Gemeinsame Patentklassifikation (CPC): (Forts.)
A61B 5/021; A61B 5/375; A61B 5/4839; A61B 5/6803; A61B 5/6817; A61B 5/6898; A61B 5/7405; A61B 5/7445; A61B 5/7455; A61M 2021/0016; A61M 2021/0022; A61M 2021/0027; A61M 2021/005;

A61M 2021/0072; A61M 2205/3306; A61M 2205/3375; A61M 2205/3553; A61M 2205/3569; A61M 2205/3584; A61M 2205/3592; A61M 2205/505; A61M 2205/507; A61M 2209/088; A61M 2230/04; A61M 2230/06; A61M 2230/10; A61M 2230/18; A61M 2230/205; A61M 2230/30; A61M 2230/432; A61M 2230/65; A61N 1/0452; A63B 22/0048; A63B 22/0076; A63B 22/0605; A63B 71/0619; A63B 2071/0625; A63B 2220/806; A63B 2220/807; A63B 2220/808; A63B 2220/836; A63B 2225/50; A63B 2230/06; A63B 2230/10; A63B 2230/30; A63B 2230/65

C-Sets
A61M 2230/04, A61M 2230/005; A61M 2230/06, A61M 2230/005; A61M 2230/10, A61M 2230/005; A61M 2230/18, A61M 2230/005; A61M 2230/205, A61M 2230/005; A61M 2230/30, A61M 2230/005; A61M 2230/432, A61M 2230/005; A61M 2230/65, A61M 2230/005

**Beschreibung**

Die Erfindung betrifft eine Vorrichtung zur neuro-vaskulären Stimulation und deren Recheneinheit.

Aus dem Stand der Technik sind Verfahren zum Gedächtnistraining bekannt. US 2001/0066005 A1 beschreibt beispielsweise ein computerimplementiertes Verfahren zum selbstgesteuerten Lernen. Neuro-physiologische Zustände des Benutzers werden für das Verfahren gemessen, um dem Benutzer eine Rückmeldung über seine Selbstregulation zu geben oder die Lernumgebung anzupassen.

Aus der WO 2015/027079 A1 ist ein System und ein Verfahren zur Verbesserung des studentischen Lernens durch Überwachen des kognitiven Zustands des Studenten bekannt. Aus der Veröffentlichung Kristina Schaaff: Enhancing mobile working memory training by using affective feedback", International Conference on Mobile Learning, 1. März 2013, Seiten 269-273, XP055375589, Lisbon, Portugal, ISBN: 978-972-893-981-6 ist ein Vorschlag zur Erweiterung des mobilen Lernens durch affektive Rückkopplung vorgeschlagen. Aus der Veröffentlichung Takahashi M. et al: "Experimental study toward mutual adaptive interface", Robot and Human Communication, 1994. RO-MAN '94 Nagoya, Japan 18-20 July 1, New York, NY, USA, IEEE, 18. Juli 1994, Seiten 271-276, XP010125417, DOI: 10.1109/ROMAN. 1994.365918, ISBN: 978-0-7803-2002-4 ist eine experimentelle Studie bezüglich eines gegenseitigen adaptiven Interface bekannt. Aus der US 2015/066104 A1 ist ein Verfahren und ein System zur Bereitstellung einer elektrischen Stimulation eines Benutzers bekannt.

Die aus dem Stand der Technik bekannten Systeme haben den Nachteil, dass diese im Wesentlichen zur Wissensvermittlung ausgelegt sind und somit eine Neuroplastizität, das heißt eine gezielte Stimulation zur Neubildung und Vernetzung von Nervenzellen im Gehirn und/oder zur Verbesserung der vaskulären Versorgung des Gehirns, wenn überhaupt nur zufällig bewirken. Die Anwendung der bekannten Systeme zur Prävention oder Behandlung von Demenz ist mit bekannten Systemen nicht möglich.

Aufgabe der Erfindung ist es daher, eine verbesserte Vorrichtung und eine Recheneinheit der Vorrichtung zur Verfügung zu stellen, die vorzugsweise die genannten Nachteile des Standes der Technik überkommt.

Die Aufgabe wird erfindungsgemäß gelöst mittels einer Vorrichtung zur neuro-vaskulären Stimulation nach Anspruch 1. Weitere vorteilhafte Ausgestaltungen sind aus der nachfolgenden Beschreibung, den Figuren sowie dem Unteranspruch zu entnehmen. Die einzelnen Merkmale der beschriebenen Ausgestaltungen sind jedoch nicht auf diese beschränkt, sondern können untereinander und mit anderen Merkmalen zu weiteren Ausgestaltungen verknüpft werden.

Es wird eine Vorrichtung zur neuro-vaskulären Stimulation zumindest umfassend zumindest einen Gehirnaktivitätssensor, zumindest einen kardio-vaskulären Sensor, zumindest eine Recheneinheit und zumindest eine Ausgabeeinheit vorgeschlagen. Die Recheneinheit weist zumindest einen Aufgabenalgorithmus auf, wobei mittels der Recheneinheit Signale zumindest des Gehirnaktivitätssensors und des kardio-vaskulären Sensors empfangbar sind und eine Aufgabe, die in Korrelation mit zumindest den Signalen zumindest des Gehirnaktivitätssensors und den Signalen des kardio-vaskulären Sensors steht, mittels des Aufgabenalgorithmus' ermittelbar und mittels der Ausgabeeinheit ausgebbar ist.

Der Begriff neuro-vaskulären Stimulation im Sinne der Erfindung umfasst eine systematische Stimulation unter Nutzung kognitiver und physiologischer Prinzipien die tierexperimentell bekanntermaßen eine Bildung und Vernetzung von Nervenzellen insbesondere aus neuronalen Stamm- oder Vorläuferzellen fördern, die Vernetzung existierender maturierter Nervenzellen verbessern und eine Blutversorgung bestimmter Gehirnregionen durch vaskuläre Plastizitätsprozesse verbessern. Bevorzugt wird die regionale Verteilung der vaskulären Plastizität im Gehirn in Verbindung mit einer neuronalen Aktivität gesteuert, geregelt und/oder angeregt, um diese in Verbindung mit einer Neuroplastizitätplastizität anzuregen.

Unter dem Begriff Gehirnaktivitätssensor ist im Sinne der vorgeschlagenen Erfindung ein Sensor oder eine Messvorrichtung zum Ermitteln einer Gehirnaktivität zu verstehen. Der Gehirnaktivitätssensor umfasst EEG-Elektroden und einen Sensor zur Messung einer Durchblutung des Gehirns.

Weiterhin bevorzugt umfasst der Gehirnaktivitätssensor zumindest eine Innenohr-EEG-Elektrode, wie beispielsweise durch die EP 2 388 680 A1 offenbart wird. Auf die EP 2 388 680 A1 wird im Rahmen dieser Offenbarung vollständig verwiesen. Weiter bevorzugt ist vorgesehen, dass der Gehirnaktivitätssensor Kopfhörer und/oder ein Headset umfasst. Weiter bevorzugt ist vorgesehen, dass der Gehirnaktivitätssensor Mittel zum Halten oder Befestigen zumindest eines Sensors und/oder einer Elektrode an einem Kopf eines Benutzers umfasst. Erfindungsgemäß ist vorgesehen, dass die Vorrichtung eine Anzahl von Gehirnaktivitätssensoren umfasst, unter anderem eine Anzahl von EEG-Elektroden. Besonders bevorzugt ist vorgesehen, dass die Vorrichtung zumindest eine Referenzelektrode für zumindest eine EEG-Elektrode umfasst.

In einer weiteren Ausgestaltung ist vorgesehen, dass der Gehirnaktivitätssensor zumindest einen Sensor zur Analyse von Augenbewegungen, Pupillometrie, ein Sensor zur Messung einer Durchblutung des Gehirns, vorzugsweise des Cortex und/oder eine Messung des autonomen Nervensystems umfasst. Beispielsweise ist ein Gehirnaktivitätssensor als Nahinfrarotspektroskopie-Sensor ausgestaltet.

Im Rahmen der Beschreibung der Erfindung wird Elektroenzephalographie mit EEG abgekürzt.

Unter dem Begriff kardio-vaskulären Sensor ist ein Sensor zu verstehen, mittels dem Puls, Herzschlag und/oder Blutdruck messbar ist. Beispielsweise umfasst eine Pulsuhr oder ein Brustgurt den kardio-vaskulären Sensor. In einer weiteren Ausgestaltung ist vorgesehen, dass der kardio-vaskuläre Sensor einen Sensor für ein Elektrokardiogramm und/oder einen Blutdrucksensor umfasst. In einer Ausgestaltung ist vorgesehen, dass zumindest zwei kardio-vaskulären Sensoren vorgesehen sind, die insbesondere an einem Hals oder einer Schläfe und an einem Hand- oder Fußgelenk anordbar sind. Aus einer Differenz oder einer Relation von mittels der Sensoren gewonnenen Pulsdaten können weitere Daten, beispielsweise ein Blutdruck ermittelt werden.

Beispielhafte Aufzählungen im Rahmen der Beschreibung der Erfindung sind nicht als abschließend anzusehen.

Die Recheneinheit kann beispielsweise ein Computer, ein Handy insbesondere ein Smartphone, ein Tablet-Computer und/oder ein Mikrocomputer sein. In einer Ausgestaltung ist vorgesehen, dass die Recheneinheit ein Server umfasst, der vorzugsweise in ein Netzwerk eingebunden ist, auf den die Vorrichtung Zugriff hat. Weiter bevorzugt weist die Vorrichtung ein Verbindungsmittel zu dem Netzwerk auf, in das die Recheneinheit eingebunden ist. In einer Ausgestaltung ist das Verbindungsmittel ein internetfähiges Mobilgerät, beispielsweise ein Handy.

Vorzugsweise ist die Recheneinheit mit einem Netzwerk, beispielsweise dem Internet verbunden. In einer weiteren Ausgestaltung ist die Recheneinheit direkt mit weiteren Komponenten der Vorrichtung verbunden. Weiter bevorzugt ist vorgesehen, dass die Recheneinheit eine Smartwatch umfasst. Insbesondere umfasst die Smartwatch zumindest einen Sensor, zumindest einen Aktuator - wie beispielsweise ein Vibrationsmotor und insbesondere eine Computerfunktionalität und/oder Verbindung zu einem Computer.

Die Ausgabeeinheit umfasst beispielsweise einen Monitor, ein Display, eine am Kopf befestigte Anzeige - wie beispielsweise eine Videobrille oder eine Virtual-Reality-Brille und/oder eine Vorrichtung zum Anzeigen einer erweiterten Realität wie beispielsweise eine Augmented-Reality-Brille. In einer weiteren Ausgestaltung ist vorgesehen, dass die Ausgabeeinheit eine Audioausgabe umfasst, beispielsweise ein Kopfhörer und/oder einen Lautsprecher.

Weiterhin ist in einer Ausgestaltung vorgesehen, dass die Ausgabeeinheit eine sensorische Ausgabe umfasst, beispielsweise mittels eines Vibrationsmotors. In einer weiteren Ausführungsform ist vorgesehen, dass die Ausgabeeinheit eine Steuerung für ein Trainingsgerät, beispielsweise eines Cardiogerätes, aufweist, bevorzugt sind Parameter des Trainingsgerätes steuerbar. Insbesondere ist ein Leistungswiderstand und/oder ein weiterer Parameter des Trainingsgerätes mittels einer Ausgabe der Vorrichtung steuerbar.

In einer weiteren Ausgestaltung ist vorgesehen, dass die Ausgabeeinheit eine Steuerung für ein Gerät zur elektrischen Muskelstimulation aufweist. In einer weiteren Ausgestaltung ist vorgesehen, dass die Ausgabeeinheit ein Mittel zur Ausgabe von Gerüchen und/oder Gesichtsausdrucksaufnahmen aufweist.

Lange Zeit galt die Vorstellung dass eine Neuroplastizität, das heißt eine Stimulation zur Neubildung und/oder Vernetzung von Nervenzellen im Gehirn, nicht möglich sei. Die aus dem Stand der Technik bekannten Vorrichtungen zum sogenannten Gehirntraining zielen daher ausschließlich auf spezifische Lerneffektverbesserung ab. Die erfindungsgemäße Vorrichtung trägt der Erkenntnis Rechnung, dass im adulten Gehirn neuronale Stammzellen vorhanden sind, die eine Neubildung von Neuronen und deren Vernetzung ermöglichen. Neu gebildete Neuronen, die nicht umgehend vernetzt werden, sterben wieder ab und haben keinen langfristigen Effekt, beispielsweise für die Behandlung oder Prävention von Demenzerkrankungen. Die Neuroplastizität geschieht jedoch bevorzugt unter bestimmten Bedingungen, die mittels der Vorrichtung vorteilhaft ermittelbar und/oder erzeugbar sind. Besonders vorteilhaft ist eine bestimmte Kardiobelastung sowie eine bestimmte Gehirnaktivität, die vorteilhaft zusammen auftreten, für die Neuroplastizität hilfreich oder notwendig.

Erfindungsgemäß ermittelt die Vorrichtung zumindest anhand der gemessenen Signale mittels eines Aufgabenalgorithmus eine Aufgabe, die dem Benutzer mittels der Ausgabeeinheit gestellt wird. Die Aufgabe wird derart ausgesucht, dass diese zu den ermittelten Signalen in Korrelation steht.

Die Aufgabe wird insbesondere derart mittels des Aufgabenalgorithmus ausgewählt, dass das Gehirn des Benutzers durch Beschäftigung mit der Aufgabe angeregt wird, insbesondere unter dem Einfluss der Kardiobelastung neue Neuronen zu bilden und weiter bevorzugt diese neuen Neuronen weiter bevorzugt zusätzlich die bereits ausgereiften existierenden Neuronen insbesondere miteinander zu vernetzen. Die Aufgabe ist bevorzugt an den Benutzer angepasst. In einer besonders bevorzugten Ausgestaltung ist vorgesehen, dass die Aufgabe derart ausgewählt ist, dass der Benutzer die Aufgabe lösen kann.

In einer weiteren Ausgestaltung ist vorgesehen, dass der Aufgabenalgorithmus zumindest eine Beantwortung der Aufgabe durch den Benutzer in die Ermittlung einer weiteren Aufgabe einbezieht, insbesondere berücksichtigt der Aufgabenalgorithmus ob der Benutzer eine vorhergehende Aufgabe richtig oder falsch beantwortet hat. Weiterhin bevorzugt ist vorgesehen, dass der Aufgabenalgorithmus eine Zeit berücksichtigt ist, die zwischen Ausgabe der vorhergehenden Aufgabe und einer Eingabe der Beantwortung verstrichen ist.

Die Aufgabe ist in einer Ausgestaltung aus unterschiedlichen Aufgabenkomplexen ausgesucht. Ein Aufgabenkomplex ist zumindest ausgewählt aus einer Gruppe umfassend mnemonische Aufgaben, ein Stroop-Test, eine Leseaufgabe, eine Melodieerkennung, eine Koordinationsaufgabe, eine Mustererkennung, eine Mustertrennung, eine Mustervervollständigung, eine Gedächtnisaufgabe - insbesondere eine Aufgabe die das prozedurale und/oder das deklarative, bevorzugt das episodische Gedächtnis anspricht, eine Aufgabe, die ein präzises Enkodieren erfordert, eine Neuheitsdetektion, eine einfache Verkettung, eine Vorwärtsverkettung - insbesondere eine transitive Inferenz, eine Generalisierung, ein salienter Reiz - insbesondere ein emotional salienter Reiz, eine Aufgabe mit eigenen Stimuli und/oder eine Aufforderung sich mehr oder sich weniger anzustrengen - beispielsweise langsamer oder schneller zu laufen, eine Navigationsaufgabe, eine multi-tasking Aufgabe mit Anforderungen an eine exekutive Kontrolle, eine Arbeitsgedächtnisaufgabe und/oder eine Aufgabe zur visuellen oder auditorischen Diskrimination ähnlicher Stimuli.

Eigene Stimuli sind in einer Ausgestaltung Fotos oder Videos von Situationen, Orten oder Personen, die der Benutzer kennt.

Erfindungsgemäß ist vorgesehen, dass der Benutzer zumindest einem Reiz ausgesetzt wird, wie beispielsweise einem Geruch, einem Bild, einer Farbe, einem Muster, einem sensitiven Reiz, einer Melodie und/oder einem Geräusch.

Ebenfalls erfindungsgemäß vorgesehen ist, dass die Aufgabe aus zumindest einem Reiz ausgesucht ist. Insbesondere ist vorgesehen, dass die Aufgabe zumindest ein Reiz ist. In einer weiteren Ausgestaltung ist vorgesehen, dass ein oder mehrere Aufgaben aus einem oder mehreren Aufgabenkomplexen kombinierbar sind. In einer weiteren Ausführungsform ist vorgesehen, dass eine Anzahl von Reizen kombinierbar sind. In einer weiteren Ausgestaltung ist vorgesehen, dass zumindest ein Reiz mit zumindest einer Aufgabe aus zumindest einem Aufgabenkomplex kombinierbar sind. In einer weiteren Ausgestaltung werden unerwartete oder deviante Reize passiv präsentiert. In einer weiteren Ausgestaltung werden unerwartete und/oder deviante Reize präsentiert und müssen aktiv detektiert werden. In einer weiteren Ausgestaltung werden Reiz- Belohnungsassoziationen im Sinne eines Verstärkungslernens gelernt und flexibel umgelernt, dies wird reversal learning genannt.

Beispielsweise sieht eine Ausführungsform vor, dass die Ausgabe zumindest einer Aufgabe mit eigenen Stimuli beziehungsweise Reize ein insbesondere zufälliges Aufnehmen von Fotos vor der Benutzung der Vorrichtung umfasst. In einer Ausgestaltung ist vorgesehen, dass eine insbesondere vom Benutzer getragene Kamera vorzugsweise automatisch über einen Zeitraum vor der Benutzung der Vorrichtung Aufnahmen macht, die bei der Benutzung der Vorrichtung gezeigt werden. Insbesondere kann eine Aufgabenstellung eine zeitliche Sortierung der Bilder umfassen. In einer weiteren Ausführungsform ist vorgesehen, dass die Bilder und weitere nicht von der Kamera des Benutzers aufgenommene Bilder gezeigt werden.

Besonders vorteilhaft ist eine Verwendung der erfindungsgemäßen Vorrichtung in der Demenz-Prävention und/oder der Demenz-Behandlung vorgesehen. In einer Ausgestaltung ist vorgesehen, dass der Aufgabenalgorithmus die Art der Demenzerkrankung des Benutzers in eine Ermittlung oder Generierung der Aufgabe einbezieht. In einer weiteren Ausgestaltung ist vorgesehen, dass die Vorrichtung zur Steigerung einer Gehirnleistungsfähigkeit verwendbar ist. Eine Ausführungsform sieht vor, dass insbesondere der Benutzer eine Zielsetzung für die Gehirnleistungsfähigkeit vorgeben kann. Eine Zielsetzung umfasst beispielsweise ein Ziel ausgewählt aus einer Gruppe umfassend eine Gedächtnisverbesserung - insbesondere eine Verbesserung des deklarativen Gedächtnisses, eine Mustererkennungsverbesserung, eine Mustertrennungsverbesserung, eine Orientierungsverbesserung, eine Verbesserung der Wahrnehmung (Perzeption), eine Sprachverbesserung, eine Konzentrationsverbesserung und/oder eine Logikverbesserung. Die Zielsetzung ist vorzugsweise mittels eines Eingabegeräts in die Vorrichtung eingebbar.

In einer Ausführungsform ist vorgesehen, dass die Vorrichtung zumindest ein Eingabegerät umfasst. Insbesondere umfasst ein Eingabegerät zumindest ein Eingabemittel ausgewählt aus einer Gruppe umfassend einen Joystick, eine Tastatur, ein Touchpad, ein Gehirnsignalfeedback, ein Mittel zur Ermittlung der Augenbewegung - Eyetracker, ein Gyroskop, ein Gehirnsignal, ein Mikrofon und/oder ein Taster beziehungsweise ein Buzzer.

In einer Ausgestaltung ist vorgesehen, dass Benutzerdaten, die Zielsetzung und/oder eine Beantwortung der gestellten Aufgabe mittels des Eingabegeräts eingebbar ist.

Benutzerdaten umfassen beispielsweise Alter, Geschlecht, Erkrankung, Medikamentation und/oder weitere Daten des Benutzers. Bevorzugt weisen Benutzerdaten Pulsdaten und/oder Neuronaldaten auf, die insbesondere bei der Benutzung der Vorrichtung ermittelbar sind. In einer weiteren Ausgestaltung weisen Benutzerdaten bei der Benutzung der Vorrichtung ermittelte, gemessene und/oder eingegebene Daten auf. In einer Ausgestaltung ist vorgesehen, dass die Benutzerdaten in den Aufgabenalgorithmus einbezogen werden, um die Aufgabe zu ermitteln.

Erfindungsgemäß ist vorgesehen, dass die Vorrichtung ein Cardiogerät umfasst. Ein Cardiogerät ist vorzugsweise ein Gerät ausgewählt aus einer Gruppe zumindest umfassend ein Laufband, ein Crosstrainer, ein Rudergerät, ein Ergometer, ein Stepper, ein Bauchtrainer, omidirektionales Laufband und/oder ein Spinninggerät. In einer weiteren Ausgestaltung weist die Vorrichtung zusätzlich ein Elektrostimulationsgerät auf. Vorteilhafterweise ist die Vorrichtung derart ausgestaltet, dass diese das Cardiogerät und optional das Elektrostimulationsgerät derart ansteuert, dass der Benutzer insbesondere eine Pulsfrequenz erreicht und vorzugsweise über einen Zeitraum hält, die optimal zur Neuroplastizität ist.

In einer Ausgestaltung weist die Vorrichtung zusätzlich einen Spirometer auf, mit dem die Einstellung des optimalen Trainingsniveaus an Hand der C02 Konzentration in der Atem luft genau angepasst werden kann. Weiter bevorzugt steuert die Vorrichtung das Cardiogerät und/oder das Elektrostimulationsgerät derart an, dass der Benutzer insbesondere durch Anstrengung oder Entspannung insbesondere eine Gehirnaktivität erreicht, die im Wesentlichen optimal zur Neuroplastizität ist.

Weiter bevorzugt steuert die Vorrichtung das Cardiogerät und/oder das Elektrostimulationsgerät derart an, dass ein Benutzer eine für die gemessene Gehirnaktivität zur Neuroplastizität optimale Herz- beziehungsweise Pulsfrequenz und/oder C02-Konzentration in der Atem luft erreicht. In einer weiteren Ausgestaltung ist vorgesehen, dass die Vorrichtung das Cardiogerät und/oder das Elektrostimulationsgerät unter Berücksichtigung der Benutzerdaten ansteuert.

Im Sinne der Erfindung ist unter einer Steuerung auch eine Regelung zu verstehen.

Besonders bevorzugt sind mittels der Recheneinheit aus den Signalen des Gehirnaktivitätssensors Neuronaldaten generierbar. Weiter bevorzugt sind mittels der Recheneinheit aus den Signalen des kardio-vaskulären Sensors Pulsdaten generierbar. Eine weitere Ausführungsform sieht vor, dass mittels des Aufgabenalgorithmus zumindest Neuronaldaten aus den Signalen des Gehirnaktivitätssensors und Pulsdaten aus den Signalen des kardio-vaskulären Sensors in Bezug zueinander setzbar sind.

In einer weiteren Ausgestaltung ist vorgesehen, dass eine automatisierte Gabe von Medikamenten erfolgt. In einer Ausführungsform weist die Vorrichtung eine Vorrichtung zur Verabreichung von Medikamenten auf. In einer Ausgestaltung ist vorgesehen, dass Medikamente in Abhängigkeit der Neuronaldaten und/oder der Pulsdaten gegebenenfalls unter Berücksichtigung der Benutzerdaten verabreichbar sind.

Weiterhin wird ein Verfahren zur Steuerung einer Vorrichtung zur neuro-vaskulären Stimulation vorgeschlagen. Die Vorrichtung umfasst zumindest ein Gehirnaktivitätssensor, zumindest einen kardio-vaskulärer Sensor, zumindest eine Recheneinheit und zumindest eine Ausgabeeinheit. Die Recheneinheit weist zumindest einen Aufgabenalgorithmus auf, wobei mittels der Recheneinheit Signale zumindest des Gehirnaktivitätssensors und des kardio-vaskulären Sensors empfangen werden und zumindest eine Aufgabe, die in Korrelation mit zumindest den Signalen zumindest des Gehirnaktivitätssensors und den Signalen des kardio-vaskulären Sensors steht, mittels des Aufgabenalgorithmus ermittelt und mittels der Ausgabeeinheit ausgegeben wird.

Der Aufgabenalgorithmus ermittelt die Aufgabe. In einer weiter bevorzugten Ausgestaltung ermittelt der Aufgabenalgorithmus eine Anzahl von Aufgaben. Bevorzugt ermittelt der Aufgabenalgorithmus immer wieder neue Aufgaben. Weiterhin bevorzugt ermittelt der Aufgabenalgorithmus eine weitere Aufgabe, nachdem der Benutzer eine Aufgabe beantwortet hat oder eine vorgegebene Zeit seit der Ausgabe der letzten Aufgabe verstrichen ist. In einer weiteren Ausgestaltung ist vorgesehen, dass eine neue Aufgabe ermittelt und vorzugsweise ausgegeben wird, wenn beispielsweise aus den Neuronaldaten erkennbar ist, dass sich der Benutzer nicht mehr mit der Aufgabe beschäftigt. Die Aufgabe wird vorzugsweise immer in Bezug zu den aktuellen Neuronaldaten und Pulsdaten ermittelt.

Weiter bevorzugt ist vorgesehen, dass der Aufgabenalgorithmus unterschiedliche Werte, beispielsweise eine Anzahl sensorisch ermittelter Werte und/oder eine Anzahl Benutzerdaten, in Relation zueinander setzt, um eine Aufgabe zu ermitteln. Weiter bevorzugt umfasst der Aufgabenalgorithmus zumindest eine Tabelle mit Aufgaben oder Aufgabenkomplexen, aus der der Aufgabenalgorithmus eine Aufgabe auswählt. In einer besonders bevorzugten Ausgestaltung ist der Aufgabenalgorithmus eine Software. Weiterhin bevorzugt ist vorgesehen, dass der Aufgabenalgorithmus eine Aufgabe aus einer Anzahl von Aufgaben, die auf der Rechnereinheit hinterlegt sind, ermittelt. Weiter bevorzugt umfasst die Rechnereinheit zumindest eine Tabelle auf die der Aufgabenalgorithmus zugreift. In einer Ausführungsform ist vorgesehen, dass der Aufgabenalgorithmus eine Funktion umfasst, die eine Aufgabe in Abhängigkeit eingegebener und/oder gemessener Werte ausgibt. In einer Ausführungsform ist vorgesehen, dass mittels des Aufgabenalgorithmus' zumindest eine Aufgabe aus zumindest einem Aufgabenkomplex ausgewählt wird. Weiter bevorzugt passt der Aufgabenalgorithmus die Aufgabe an die Benutzerdaten an.

In einer weiteren Ausgestaltung ist vorgesehen, dass Neuronaldaten aus den Signalen des Gehirnaktivitätssensors ermittelt werden. Vorzugsweise wird zumindest ein Frequenzband eines EEG ermittelt beziehungsweise dessen Vorliegen überprüft. Weiter bevorzugt wird eine Amplitude zumindest eines Frequenzbandes ermittelt. In einer Ausführungsform ist vorgesehen, dass eine Abweichung einer optimalen Gehirnaktivität für die Neuroplastizität des Benutzers ermittelt wird. Weiter bevorzugt ist vorgesehen, dass eine Abweichung von einem EEG-Frequenzband, das optimal für die Neuroplastizität des Benutzers ist, ermittelt wird.

In einer Ausführungsform ist vorgesehen, dass eine Abtastrate der Signale am Gehirnaktivitätssensor von etwa 1 Hz bis etwa 600 Hz, bevorzugt etwa 2 bis etwa 300 Hz verwendet wird. In einer Ausgestaltung ist vorgesehen, dass EEG-Frequenzbänder von Delta bis Gamma ermittelt werden. Bevorzugt wird zumindest das Delta-Frequenzband von etwa 0,5 Hz bis etwa 4 Hz ermittelt. Weiter bevorzugt wird zumindest das Theta-1 -Frequenzband von etwa 4 Hz bis etwa 6,5 Hz ermittelt. Weiter bevorzugt wird zumindest das Theta-2-Frequenzband von etwa 6,5 Hz bis etwa 8 Hz ermittelt. Weiter bevorzugt wird zumindest das Alpha-Frequenzband von etwa 8 Hz bis etwa 13 Hz ermittelt.

Weiter bevorzugt wird zumindest das niedrige Beta-Frequenzband von etwa 13 Hz bis etwa 15 Hz ermittelt. Weiter bevorzugt wird zumindest das mittlere Beta-Frequenzband von etwa 15 Hz bis etwa 21 Hz ermittelt. Weiter bevorzugt wird zumindest das hohe Beta-Frequenzband von etwa 21 Hz bis etwa 30 Hz ermittelt. Weiter bevorzugt wird zumindest das Gamma-Frequenzband von etwa 30 Hz bis etwa 80 Hz ermittelt.

In einer Ausgestaltung ist vorgesehen, dass dem Benutzer in einem Enkodierungs-Modus zumindest eine Lernaufgabe mittels der Vorrichtung gestellt wird. Die Lernaufgabe kann beispielsweise eine Abfolge von Bildern sein, die sich der Benutzer einprägen soll. Besonders vorteilhaft ist vorgesehen, dass die Vorrichtung die Lernaufgabe erst stellt, wenn die Vorrichtung eine Erhöhung der Amplitude in einem Frequenzband von etwa 3 Hz bis etwa 8 Hz, bevorzugt etwa 4 bis etwa 8 Hz, weiter bevorzugt des Theta-1 -Frequenzbandes und/oder Theta-2-Frequenzbandes ermittelt, vorzugsweise eine Erhöhung der Amplitude um zumindest etwa 10 %, weiter bevorzugt zumindest etwa 20 % gegenüber einer vorher ermittelten Referenzamplitude, weiter bevorzugt bei einer Amplitude von über etwa 20 $\mu$V, weiter bevorzugt über etwa 50 $\mu$V, weiter bevorzugt über etwa 70 $\mu$V.

In einer weiteren Ausgestaltung wird eine benutzerspezifische Referenzamplitude und eine benutzerspezifische Überschreitung der Referenzamplitude als Schwellwert zur Auslösung der Ermittlung der Aufgabe im Vorfeld ermittelt oder durch die Benutzung der Vorrichtung ermittelt und/oder angepasst. Die Referenzamplitude wird gemäß einer Ausgestaltung in einer Ruhephase, beispielsweise im Schlaf oder wenn der Benutzer etwa seinen Ruhepuls aufweist, oder in einer weiteren Ausgestaltung beim Bearbeiten einer Aufgabe ermittelt. Zur Berechnung der Referenzamplitude wird vorzugsweise ein Amplituden-Mittelwert über einen Zeitraum von wenige Millisekunden, beispielsweise etwa 10 ms bis etwa 90 ms bis mehrere Minuten, beispielsweise etwa 2 min. bis etwa 15 min. Die Referenzamplitude kann gemäß einer Ausgestaltung als konstante Größe über mehrere Anwendungen hinweg genutzt werden oder gemäß einer weiteren Ausgestaltung dynamisch von Anwendung zu Anwendung und/oder innerhalb einer Anwendung mehrmals neu bestimmt werden.

In einer weiteren Ausgestaltung ist vorgesehen, dass in einem Abruf-Modus eine Erinnerungsaufgabe gestellt wird. Vorzugsweise umfasst die Erinnerungsaufgabe das Erinnern von zumindest Teilen der vorher gestellten Lernaufgabe. Weiterhin bevorzugt ist vorgesehen, dass die Vorrichtung die Erinnerungsaufgabe erst stellt, wenn die Vorrichtung eine Erhöhung der Amplitude in einem Frequenzband von etwa 3 Hz bis etwa 8 Hz, bevorzugt etwa 4 bis etwa 8 Hz, weiter bevorzugt des Theta-1 - Frequenzbandes und/oder Theta-2-Frequenzbandes ermittelt, vorzugsweise eine Erhöhung der Amplitude um zumindest etwa 10 %, weiter bevorzugt zumindest etwa 20 % gegenüber einer vorher ermittelten Referenzamplitude, weiter bevorzugt bei einer Amplitude von über etwa 20 $\mu$V, weiter bevorzugt über etwa 50 $\mu$V, weiter bevorzugt über etwa 70 $\mu$V. In einer weiteren Ausgestaltung wird eine benutzerspezifische Referenzamplitude und eine benutzerspezifische Überschreitung der Referenzamplitude als Schwellwert zur Auslösung der Ermittlung der Aufgabe im Vorfeld ermittelt oder durch die Benutzung der Vorrichtung ermittelt und/oder angepasst.

In einer weiteren Ausführungsform ist vorgesehen, dass insbesondere während eine Aufgabe ausgegeben wird, dass zumindest das Alpha-Frequenzband und/oder das Beta-Frequenzband ermittelt wird. Weiterhin bevorzugt ist vorgesehen, dass bei einer Änderung des Alpha-Frequenzbandes und/oder des Beta-Frequenzbandes die Aufgabe angepasst wird. Wird beispielsweise anhand einer Änderung eines Frequenzbandes, vorzugsweise des Alpha-Frequenzbandes und/oder des Beta-Frequenzbandes, ermittelt, dass eine insbesondere sublimale Frustration - das heißt eine Frustationsreaktion unter der Wahrnehmungsschwelle des Benutzers, Überforderung und/oder Bestrafungsreaktion stattfindet, wird die Aufgabe angepasst. Beispielsweise wird eine Abfolge von zu erinnernden Bildern verlangsamt und/oder die Frequenz der Abfolge nicht beschleunigt.

Weiterhin wird die Aufgabe in einer Ausgestaltung derart gestellt und/oder angepasst, dass eine Belohnungsreaktion, insbesondere eine Ausschüttung von Endorphinen, ausgelöst wird. Weiterhin wird die Aufgabe in einer Ausgestaltung derart gestellt und/oder angepasst, dass ein Gehirnsignal, wie es für eine Belohnungserwartung und/oder Belohnungsvorhersagefehler typisch ist, gemessen wird. Vorteilhafterweise wird die Aufgabe anhand des Gehirnsignals geregelt. Insbesondere wird die Aufgabe insbesondere durch die Neuronaldaten derart angepasst, dass der Benutzer diese lösen kann.

In einer weiteren Ausgestaltung ist vorgesehen, dass mittels einer Nahinfrarotspektroskopie eine Belohnungsreaktion und/oder eine insbesondere sublimale Frustration, Überforderung und/oder Bestrafungsreaktion ermittelt wird. Beispielsweise wird eine Nahinfrarotspektroskopie zumindest einem Bereich zumindest eines Schläfenlappens durchgeführt.

In einer weiteren Ausgestaltung ist vorgesehen, dass eine Überwachung und/oder Speicherung der Signale vom Gehirnaktivitätssensor oder der Neuronaldaten erfolgt.

Weiterhin ist in einer Ausführungsform vorgesehen, dass eine Speicherung der Signale des kardio-vaskulären Sensors oder der Pulsdaten erfolgt. Bevorzugt ist vorgesehen, dass Benutzerdaten anhand der Neuronaldaten und/oder der Pulsdaten ermittelt werden.

Beispielsweise ist eine Anpassung der Aufgabe anhand von Benutzerdaten vorgesehen, die Pulsdaten und/oder Neuronaldaten zumindest einer vorherigen Messung beziehungsweise Benutzung der Vorrichtung beinhalten. In einer weiteren Ausgestaltung ist vorgesehen, dass eine Anpassung der Aufgabe in situ, das heißt während der Benutzung der Vorrichtung erfolgt. In einer weiteren Ausgestaltung ist vorgesehen, dass die Vorrichtung eine Diagnostik insbesondere von Demenzerkrankungen unterstützt und/oder automatisiert durchführt. In einer weiteren Ausführungsform ist vorgesehen, dass anhand der ermittelten Benutzerdaten eine Diagnostik, eine Medikamentation und/oder eine Medikamtationsempfehlung erfolgt.

In einer weiteren Ausgestaltung ist vorgesehen, dass eine Amplitude eines evozierten Potentials ermittelt wird.

Vorzugsweise wird ein evoziertes Potential nach einer visuellen, auditorischen, olfaktorischen, gustatorischen und/oder taktilen Informationspräsentation ermittelt, bevorzugt etwa 10 ms bis etwa 3000 ms, weiter bevorzugt etwa 100 ms bis etwa 1000 ms nach Beginn der Informationspräsentation. Weiterhin bevorzugt ist vorgesehen, dass mittels des ermittelten evozierten Potentials eine Diskrimination neuer und bekannter Information erfolgt. Beispielsweise kann mittels der evozierten Potentiale ermittelt werden, ob der Benutzer sich an eine Information erinnert, bevorzugt unabhängig davon ob der Benutzer dies bewusst wahrnimmt. Bevorzugt wird die Aufgabe anhand zumindest des ermittelten evozierten Potentials bevorzugt unter Berücksichtigung der Informationspräsentation angepasst.

In einer weiteren Ausgestaltung ist vorgesehen, dass die Amplitude des Alpha- Frequenzbandes insbesondere in einem Zeitfenster von etwa 100 ms bis etwa 3000 ms, weiter bevorzugt etwa 100 ms bis etwa 1000 ms nach Beginn der Informationspräsentation ermittelt wird. Weiterhin bevorzugt ist vorgesehen, dass mittels der Amplitude des Alpha-Frequenzbandes eine Diskrimination neuer und bekannter Information erfolgt. Bevorzugt wird die Aufgabe anhand des zumindest der Amplitude des Alpha-Frequenzbandes bevorzugt unter Berücksichtigung der Informationspräsentation angepasst.

In einer weiteren Ausgestaltung ist vorgesehen, dass die Amplitude des Theta-1 - Frequenzbandes insbesondere in einem Zeitfenster von etwa 100 ms bis etwa 3000 ms, weiter bevorzugt etwa 100 ms bis etwa 1000 ms nach Beginn der Informationspräsentation ermittelt wird. Weiterhin bevorzugt ist vorgesehen, dass mittels der Amplitude des Theta-1 -Frequenzbandes eine Diskrimination neuer und bekannter Information erfolgt. Bevorzugt wird die Aufgabe anhand des zumindest der Amplitude des Theta-1 -Frequenzbandes bevorzugt unter Berücksichtigung der Informationspräsentation angepasst.

In einer weiteren Ausgestaltung ist vorgesehen, dass die Amplitude des Theta-2-Frequenzbandes insbesondere in einem Zeitfenster von etwa 100 ms bis etwa 3000 ms, weiter bevorzugt etwa 100 ms bis etwa 1000 ms nach Beginn der Informationspräsentation ermittelt wird. Weiterhin bevorzugt ist vorgesehen, dass mittels der Amplitude des Theta-2-Frequenzbandes eine Diskrimination neuer und bekannter Information erfolgt. Bevorzugt wird die Aufgabe anhand des zumindest der Amplitude des Theta-2-Frequenzbandes bevorzugt unter Berücksichtigung der Informationspräsentation angepasst.

Unter dem Begriff Information im Sinne der Erfindung ist zumindest eine visuelle, auditorische, olfaktorische, gustatorische und/oder taktile Information zu verstehen.

Evozierte Potentiale sind Potentialunterschiede in den vom Gehirnaktivitätssensor ermittelten Signalen, welche durch eine Reizung eines Sinnesorgans oder peripheren Nervs ausgelöst werden. Bevorzugt sind alle gezielt ausgelösten elektrischen Phänomene im EEG evozierte Potentiale. Vorzugsweise weisen evozierte Potentiale Amplituden von etwa 0,5 $\mu$V bis etwa 20 $\mu$V, weiter bevorzugt etwa 1 $\mu$V bis etwa 15 $\mu$V auf.

In einer weiteren Ausgestaltung ist vorgesehen, dass beispielsweise mittels Nahinfrarotspektroskopie eine Perfusion, das heißt Durchblutung, einer Gehirnregion ermittelt wird. Weiterhin bevorzugt ist vorgesehen, dass mittels ermittelten Perfusion der Gehirnregion eine Diskrimination neuer und bekannter Information erfolgt. Erfindungsgemäß wird die Aufgabe anhand des zumindest der ermittelten Perfusion der Gehirnregion bevorzugt unter Berücksichtigung der Informationspräsentation angepasst.

Erfindungsgemäß ist vorgesehen, dass Pulsdaten aus den Signalen des kardio-vaskulären Sensors ermittelt werden. Pulsdaten können beispielsweise eine Herzfrequenz, eine retrograde Pulsfrequenz, eine anterograde Pulsfrequenz, ein peripheres Pulsdefizit und/oder ein Blutdruck sein.

Es wird eine Abweichung von einem individuellen Trainingspuls ermittelt. Beispielsweise wird ein Maximalpuls des Benutzers vorzugsweise im Vorfeld der Benutzung der Vorrichtung ermittelt. Der Maximalpuls kann in Relation zum Alter des Benutzers beispielsweise durch die Formel

$$\text{Maximalpuls} = 220 - \text{Lebensalter in Jahren}$$

ermittelt werden. In einer Ausgestaltung können weitere Benutzerdaten in die Berechnung des Maximalpulses aufgenommenen werden. Bevorzugt ist vorgesehen, dass der Maximalpuls mittels einer Trainingseinheit, die der Benutzer vorzugsweise im Vorfeld der Benutzung der Vorrichtung absolviert, ermittelt. Der Trainingspuls ist der Puls, der für die Neuroplastizität im Gehirn des Benutzers optimal ist. Vorzugsweise ist der Trainingspuls in Relation zum Maximalpuls. Beispielsweise ist der Trainingspuls etwa 30 % bis etwa 70 % des Maximalpuls, bevorzugt etwa 40 % bis etwa 60 %, weiter bevorzugt etwa 40 % bis etwa 50 %. In einer weiteren vorteilhaften Ausgestaltung wird die C02-Konzentration der Atemluft und/oder der Sauerstoffgehalt im Blut gemessen. Weiterhin vorteilhaft wird ein Trainingspuls derart festgelegt, dass der Benutzer sich dabei an der anaeroben Schwelle befindet.

Wird im Rahmen der Erfindung der Begriff"etwa" verwendet, so ist darunter ein Toleranzbereich zu verstehen, den der Fachmann auf diesem Gebiet für üblich erachtet, insbesondere ist ein Toleranzbereich von $\pm 20\%$, bevorzugt $\pm 10\%$ vorgesehen. Auch der Begriff"im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen ist.

Gemäß der Erfindung ist vorgehen, dass bei einer Abweichung von der für die Neuroplastizität optimalen Gehirnaktivität der Benutzer stimuliert wird, um die für die Neuroplastizität optimale Gehirnaktivität im Wesentlichen zu erreichen. Eine Stimulation kann beispielsweise mittels einer Aufgabe erfolgen, die insbesondere mittels der Ausgabeeinheit ausgegeben wird. In einer weiteren Ausgestaltung ist vorgesehen, dass die Stimulation zumindest eine Anregung oder Aufforderung insbesondere mittels der Ausgabeeinheit an den Benutzer ist, physisch höhere oder niedrigere Leistung zu erbringen. Beispielsweise ist in einer Ausgestaltung vorgesehen, dass ein Leistungswiderstand des Cardiogerätes geändert wird. In einer weiteren Ausgestaltung ist vorgesehen, dass mittels gehirninterner, gehirnexterner und/oder extracranialer Stromimpulsen eine Änderung der Gehirnaktivität induziert wird. In einer weiteren Ausgestaltung ist vorgesehen, dass mittels gehirninterner, gehirnexterner und/oder extracranialer Magnetfeldern eine Änderung der Gehirnaktivität induziert wird. In einer weiteren Ausgestaltung ist vorgesehen, dass insbesondere eine Anzahl von Maßnahmen vorgesehen ist, um die optimale Gehirnaktivität des Benutzers für die Neuroplastizität im Wesentlichen zu erreichen.

Die Gehirnaktivität wird zumindest aus einem oder mehreren Signalen oder Daten ausgesucht aus einer Gruppe umfassend zumindest ein elektrisches Signal, bevorzugt ein EEG-Signal in Form von evozierten Potentialen und/oder gemittelte Oszillationen eines EEG-Frequenzbandes, Perfusionsänderung in zumindest einer Gehirnregion, Pupillendurchmesser und/oder Blickrichtung ermittelt werden. In einer Ausgestaltung ist vorgesehen, dass Werte für die optimale Gehirnaktivität des Benutzers für die Neuroplastizität während oder vor der Benutzung der Vorrichtung ermittelt werden, insbesondere sind die optimalen Werte, die sich während der Erwartung von Neuheit, beispielsweise neuen Bildern, neuen Gerüchen, neuen Tönen und/oder in Erwartung von Belohnung, beispielsweise Punkte in einem Spiel, einstellen. Vorzugsweise wird die optimale Gehirnaktivität spezifisch für den Benutzer ermittelt. Insbesondere wird aus den bevorzugt während der Benutzung der Vorrichtung ermittelten Benutzerdaten eine optimale Gehirnaktivität ermittelt. Beispielsweise wird eine oder mehrere Aufgaben bei unterschiedlichen Pulsdaten und Neuronaldaten gegebenenfalls wiederholt vorgenommen und die optimale Gehirnaktivität zu ermitteln.

Erfindungsgemäß ist vorgesehen, dass bei einer Abweichung des Pulses des Benutzers vom vorgegebenen Trainingspuls der Benutzer stimuliert wird, um etwa den Trainingspuls zu erreichen. Hierbei ist vorgesehen, dass die Stimulation zumindest eine Anregung oder Aufforderung insbesondere mittels der Ausgabeeinheit an den Benutzer ist, physisch höhere oder niedrigere Leistung zu erbringen. Erfindungsgemäß wird ein Leistungswiderstand des Cardiogerätes geändert. In einer weiteren Ausgestaltung ist vorgesehen, dass mittels eines Elektrostimulationsgeräts der Benutzer im Wesentlichen auf einen Trainingspuls gebracht wird.

In einer weiteren Ausgestaltung ist vorgesehen, dass der Aufgabenalgorithmus mittels zumindest eines Benutzerdatenelements die Aufgabe ermittelt. Vorzugsweise wird das zumindest eine Benutzerdatenelement in Relation zu zumindest einem Pulsdatenelement und zumindest einem Neuronaldatenelement gesetzt, um die Aufgabe zu ermitteln. In einer weiteren Ausgestaltung ist vorgesehen, dass zumindest eine Anzahl von Relationen der Pulsdaten zu den Neuronaldaten jeweils zumindest einer Aufgabe oder einem Aufgabenkomplex zugeordnet sind. Weiterhin vorteilhaft ist vorgesehen, dass eine Aufgabe erst dann von dem Aufgabenalgorithmus ermittelt wird, wenn die Relation der Pulsdaten und Neuronaldaten über eine definierte Zeit etwa konstant sind, beispielsweise etwa 10 s bis etwa 90 s, bevorzugt etwa 20 s bis etwa 60 s, weiter bevorzugt etwa 30 s bis etwa 40 s.

In einer weiteren Ausführungsform ist vorgesehen, dass eine Aufgabe mittels des Aufgabenalgorithmus' zumindest in Korrelation zu einer Eingabe mittels eines Eingabegeräts ermittelt wird. Wird beispielsweise eine nicht korrekte Eingabe als Antwort auf die Aufgabe eingegeben, wir die nächste Aufgabe leichter gestellt.

Weiterhin ist in einer Ausgestaltung vorgesehen, dass die Aufgabe mittels des Aufgabenalgorithmus' zumindest in Korrelation mit einer Zeitdifferenz zwischen einer Ausgabe der Aufgabe und der Eingabe der Antwort ermittelt wird. In einer weiteren Ausgestaltung ist vorgesehen, dass eine Aufgabe in Korrelation mit einer bestimmten Augenbewegung ermittelt wird.

Weiterhin ist in einer Ausgestaltung vorgesehen, dass die Aufgabe bei einer bestimmten Augenbewegung gestellt wird. Weiterhin ist in einer Ausgestaltung vorgesehen, dass eine Aufgabe gestellt wird, wenn bestimmte Neuronaldaten ermittelt sind.

Die Eingabe kann mittels eines Joysticks, einer Tastatur, eines Touchpads, eines Gehirnsignalfeedbacks, eines Mittels zur Ermittlung der Augenbewegung, eines Gyroskops, eines Gehirnsignals, eines Mikrofons und/oder eines Tasters erfolgen. In einer Ausgestaltung ist beispielsweise vorgesehen, dass zumindest ein Gehirnsignal, beispielsweise zumindest ein EEG-Signal verwendet wird, um die Eingabe zu tätigen.

In einer weiteren Ausgestaltung ist vorgesehen, dass der Aufgabenalgorithmus anhand der Benutzerdaten eines oder mehrerer Benutzer angepasst wird.

In einer weiteren Ausführungsform ist vorgesehen, dass eine Situation im Vorfeld der Benutzung der Vorrichtung mittels zumindest einer Kamera und/oder zumindest eines Mikrofons aufgezeichnet wird, die mittels der Ausgabeeinheit ausgegeben wird. Vorzugsweise wird eine Alltagssituation aus dem Alltag des Benutzers ausgegeben. In einer weiteren Ausgestaltung ist vorgesehen, dass eine sportliche Situation oder eine Umgebung insbesondere aus Sicht des Benutzers oder eines Dritten aufgenommen wird, die bei der Benutzung der Vorrichtung als Aufgabe, alternativ zu einer Aufgabe oder in Verbindung mit einer Aufgabe ausgegeben wird. Beispielsweise ist vorgesehen, dass ein Benutzer eine Wanderung macht und eine Kamera mitführt, die währenddessen Foto und/oder Videoaufnahmen macht. Bei der Benutzung der Vorrichtung werden die Bilder und/oder Filme eingespielt, um Erinnerungen zu stimulieren und/oder den Benutzer zu den eingeblendeten Fotos und/oder Videos vorzugsweise automatisiert durch die Vorrichtung weiter bevorzugt in Abhängigkeit zumindest der Pulsdaten und der Neuronaldaten zu befragen. In einer weiteren Ausgestaltung ist vorgesehen, dass die Fotos und/oder Videos mit einer Bewegung auf dem Cardiogerät synchronisiert werden. So kann der Benutzer vorteilhafterweise beispielsweise die Wanderstrecke virtuell ablaufen.

Eine Kamera ist bevorzugt eine Digitalkamera, beispielsweise eine CCD-Kamera. In einer weiteren Ausgestaltung ist vorgesehen, dass die Kamera eine 3D-Kamerasystem umfasst, beispielsweise eine Stereokamera, eine Kamera mit Triangulationssystem, eine Kamera mit Photomischdetektor, eine Kamera mit Interferometer und/oder eine Lichtfeldkamera. Weiterhin ist in einer Ausgestaltung vorgesehen, dass die Kamera eine Panoramakamera umfasst, beispielsweise eine sich drehende Kamera, eine Zeilenkamera, eine Kamera mit zumindest einem Weitwinkel- oder Fischaugenobjektiv und/oder eine Kamera mit Pilzspiegel. In einer bevorzugten Ausgestaltung ist vorgesehen, dass die Kamera eine 360cam der Firma Giroptic Inc. ist.

In einer weiteren Ausgestaltung ist vorgesehen, dass eine Anzahl von Vorrichtungen miteinander vernetzt ausgestaltet sind. In einer weiteren Ausgestaltung ist vorgesehen, dass zumindest zwei Benutzer die Vorrichtungen gemeinsam benutzen. Insbesondere ist in einer Ausgestaltung vorgesehen, dass die Benutzer gemeinsam und/oder gegeneinander eine Aufgabe oder eine Anzahl von Aufgaben lösen, insbesondere miteinander spielen. Die Benutzer können sich gemäß einer Ausgestaltung im Lösen der Aufgaben messen, gegeneinander antreten und/oder kooperativ betätigen. Insbesondere ist von jedem Benutzer die kardio-vaskuläre Betätigung, bevorzugt entsprechend seines Benutzerprofils, zu leisten.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen

Fig. 1 schematisch eine erste Ausgestaltung einer erfindungsgemäßen Vorrichtung; und
Fig. 2 schematische eine weitere Ausgestaltung einer erfindungsgemäßen Vorrichtung.

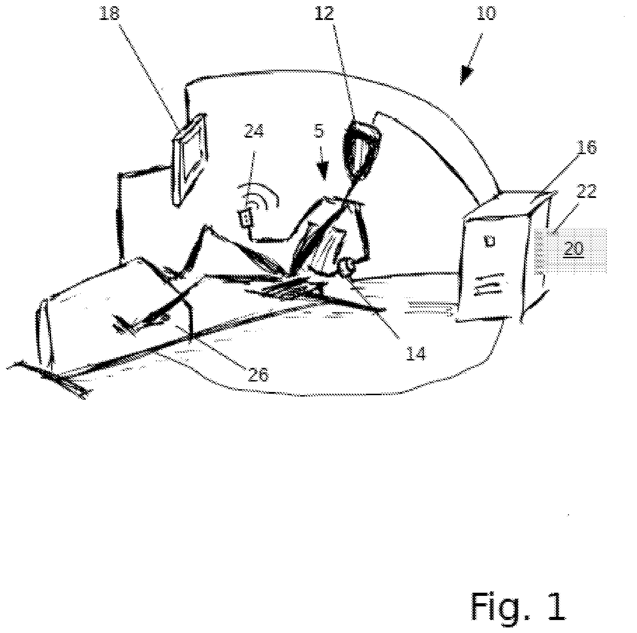

Fig. 1 zeigt schematisch eine Vorrichtung 10 zur neuro-vaskulären Stimulation. Dieser umfasst eine Anzahl von Gehirnaktivitätssensoren 10 die zur Abnahme von EEG-Signalen an einer Kopfoberfläche ausgestaltet sind. Weiterhin umfasst die Vorrichtung 10 einen kardio-vaskulären Sensor 14, der beispielsweise einen Puls am Unterarm und/oder einen Blutdruck misst. Aufgenommene Messwerte der Gehirnaktivitätssensoren 10 und des kardio-vaskulären Sensors 14 werden an eine Recheneinheit 16 übermittelt, welche die Messwerte in Neuronaldaten und Pulsdaten umwandelt. Mittels eines Aufgabenalgorithmus 20 werden zumindest die Pulsdaten und die Neuronaldaten, vorzugsweise mit weiteren Benutzerdaten in Relation zueinander gesetzt. Der Aufgabenalgorithmus 20 ermittelt hieraus eine in der Recheneinheit 16 hinterlegte Aufgabe 22, die mittels einer Ausgabeeinheit 18, die in Fig. 1 als Monitor ausgestaltet ist, ausgegeben wird. Ein Benutzer 5, der die Vorrichtung 10 benutzt, betätigt ein Cardiogerät 26, um einen Trainingspuls zu erreichen. Erfindungsgemäß steuert die Recheneinheit 16 das Cardiogerät 26 derart an, dass der Benutzer 5 den Trainingspuls im Wesentlichen erreicht und vorzugsweise im Wesentlichen hält. So steuert die Recheneinheit 16 einen Leistungswiderstand des Cardiogeräts 26. Ermittelt die Recheneinheit 16 aus den Pulsdaten, dass der für den Benutzer individuelle Trainingspuls im Wesentlichen erreicht ist, und aus den Neuronaldaten, dass im Wesentlichen eine Gehirnaktivität vorliegt, die eine Neuroplastizität begünstigt, wird über die Ausgabeeinheit 18 die mittels des Aufgabenalgorithmus 20 ermittelte Aufgabe 22 ausgegeben. Der Benutzer 5 gibt nun eine Antwort auf die Aufgabe 22 mittels eines Eingabegeräts 24 ein. Vorzugsweise übermittelt das Eingabegerät 24 die Antwort des Benutzers 5 an die Recheneinheit 16. Hiernach erfolgen in einer Ausgestaltung die Stellung zumindest einer weiteren Aufgabe 22, die vorzugsweise neu mittels des Aufgabenalgorithmus 20 aus den Pulsdaten und den Neuronaldaten ermittelt wird.

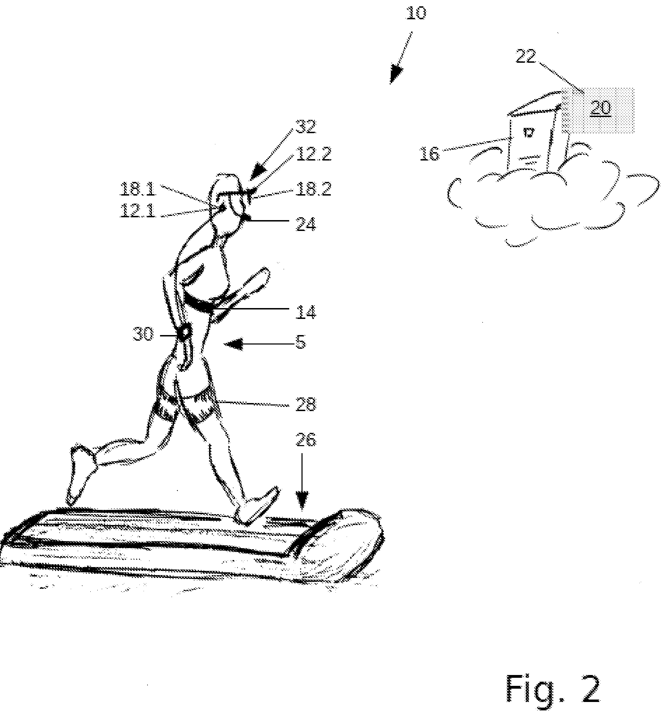

Fig. 2 zeigt schematisch eine weitere Ausgestaltung der Vorrichtung 10. Dieser umfasst einen Gehirnaktivitätssensor 12.1 , der als In-Ear-Kopfhörer ausgestaltet ist. Der In-Ear- Kopfhörer fungiert gleichzeitig als akustische Ausgabeeinheit 18.1 . Der Gehirnaktivitätssensor 12.1 ist mit einem am Arm tragbaren Computer, einer Smartwatch 30, verbunden ist. Die Smartwatch ist weiterhin kabelgebunden oder per Funkverbindung mit einem kardio-vaskulären Sensor 14 verbunden, der als Brustgurt ausgestaltet ist. Die Smartwatch 30 ist vorzugsweise über eine Funkverbindung, beispielsweise über eine Mobilfunkverbindung mit der Recheneinheit 16 verbunden, die hier beispielhaft als Cloud-Server ausgestaltet ist. Auf dem Cloud-Server liegen gemäß einer Ausgestaltung Benutzerdaten von einer Anzahl von Benutzern vor, die mittels mobilen und/oder stationären Rechnern beziehungsweise jeweils mittels einer Vorrichtung 10 insbesondere über das Internet auf den Cloud-Server

Weiterhin zeigt Fig. 2, dass der Benutzer 5 eine Brille 32 trägt, die Teil der Vorrichtung 10 ist und die vorzugsweise als Ausgabeeinheit 18.2 fungiert. Bevorzugt ist die Brille 32 eine Augmented-Reality-Brille. Weiterhin umfasst die Brille einen Sensor für eine Pupillenreaktion, der einen zusätzlichen Gehirnaktivitätssensor 18.2 darstellt. Weiterhin ist als Eingabegerät 24 ein Mikrofon vorgesehen, das in der gezeigten Ausgestaltung an der Brille 32 angeordnet ist. In einer weiteren Ausgestaltung ist das Mikrofon mit dem Kopfhörer verbunden oder als Einzelteil ausgestaltet.

Hier beispielhaft läuft der Benutzer auf einem Cardiogerät 26, das als Laufband ausgestaltet ist. In einer nicht erfindungsgemäßen Ausgestaltung ist vorgesehen, dass der Benutzer ohne ein Cardiogerät trainiert, beispielsweise joggt, geht, wandert, rudert oder eine sonstige körperliche Aktivität ausübt, die zu einer Herzfrequenzsteigerung führt. In dem gezeigten Beispiel weist die Vorrichtung ein Elektrostimulationsgerät 28 auf, das einen Trainingseffekt, insbesondere die Herzfrequenz des Benutzers, beeinflusst. Das Elektrostimulationsgerät 28 ist hier beispielhaft an Oberschenkeln des Benutzers 5 angeordnet. In einer weiteren Ausgestaltung ist vorgesehen, dass das Elektrostimulationsgerät 28 an einer oder mehreren beliebigen Stellen eines Körpers des Benutzers 5 angeordnet ist.

Beispielsweise trainiert der Benutzer 5 in einem Fitnessstudio auf einem Cardiogerät 26. Die von den Gehirnaktivitätssensoren 18.1 und/oder 18.2 ermittelten Sensorsignale sowie die von dem kardio-vaskulären Sensor 14 ermittelten Sensorsignale werden von der Smartwatch 30 in Neuronaldaten und Pulsdaten gewandelt. Über die Mobilfunkverbindung sendet die Smartwatch die Neuronaldaten und die Pulsdaten an die Recheneinheit 16, die als Cloud-Server im Internet ausgestaltet ist. Der in der Rechnereinheit 16 hinterlegte Aufgabenalgorithmus 20 ermittelt eine Aufgabe 22 aus einer Anzahl von Aufgaben, die in der Rechnereinheit 16 hinterlegt sind. Insbesondere wird die Aufgabe erst dann ermittelt, wenn Pulsdaten und Neuronaldaten gegebenenfalls unter Berücksichtigung der Benutzerdaten im Wesentlichen optimale Bedingungen für die Neuroplastizität wiedergeben. Die ermittelte Aufgabe wird an die Smartwatch 30 gesendet, um diese mittels der Ausgabeeinheit 18.1 und/oder 18.2 auszugeben. Sind noch keine optimalen Bedingungen für die Neuroplastizität mittels des Aufgabenalgorithmus erkennbar, sendet die Recheneinheit 16 in einer Ausgestaltung entweder Aufgaben, die eine Gehirnaktivität beeinflussen, und/oder Signale zur Ansteuerung des Elektrostimulationsgeräts 28. Weiterhin sieht eine Ausgestaltung vor, dass der Benutzer 5 eine Rückmeldung über seinen Neuronaldaten und/oder seine Pulsdaten erhält, die beispielsweise graphisch mittels der Ausgabeeinheit 18.2 ausgegeben werden.

Der Benutzer kann sein körperliches Training beispielsweise an den Pulsdaten orientieren. Insbesondere wird dem Benutzer vorzugsweise graphisch oder farblich angezeigt, ob er einen im Wesentlichen optimalen Trainingspuls für die Neuroplastizität aufweist. Weiterhin ist in einer Ausgestaltung vorgesehen, dass dem Benutzer auditive und/oder visuelle Reize über die Ausgabeeinheit 18.1 und/oder 18.2 insbesondere zur Stimulation der Gehirnaktivität ausgegeben werden.

Nach Ausgabe der Aufgabe beantwortet der Benutzer diese sprachlich, wobei das Eingabegerät 24 eine Spracheingabe empfängt und diese an die Smartwatch 30 beziehungsweise an die Recheneinheit 16 zur Auswertung weiter leitet. Hiernach erfolgt gegebenenfalls in einem zeitlichen Abstand eine weitere Ermittlung einer weiteren Aufgabe 22. Insbesondere wird bei der Ermittlung der neuen Aufgabe 22 zumindest eine vorhergehende Aufgabe 22 und jeweils eine Antwort auf die zumindest eine vorhergehende Aufgabe 22 berücksichtigt.

**Patentansprüche**

1. Vorrichtung (10) zur neuro-vaskulären Stimulation, zumindest umfassend zumindest einen Gehirnaktivitätssensor (12), aus dessen Signalen Neuronaldaten des Gehirns ermittelbar sind, wobei der Gehirnaktivitätssensor (12) EEG-Elektroden und einen Sensor zur Messung einer Durchblutung des Gehirns umfasst, aus dessen Signalen Durchblutungsdaten des Gehirns ermittelbar sind, zumindest einen kardio-vaskulären Sensor (14), aus dessen Signalen Puls-Daten des Benutzers ermittelbar sind, zumindest eine Recheneinheit (16) und zumindest eine Ausgabeeinheit (18), wobei die Recheneinheit (16) zumindest einen Aufgabenalgorithmus (20) aufweist, wobei mittels der Recheneinheit (16) Signale zumindest des Gehirnaktivitätssensors (12) und des kardio-vaskulären Sensors (14) empfangbar sind und eine Aufgabe (22), die in Korrelation mit zumindest den Signalen zumindest des Gehirnaktivitätssensors (12) und den Signalen des kardio-vaskulären Sensors (14) steht, mittels des Aufgabenalgorithmus' (20) ermittelbar und mittels der Ausgabeeinheit (18) an den Benutzer der Vorrichtung (10) ausgebbar ist, wobei mittels des Aufgabenalgorithmus' (20) zumindest Neuronaldaten des Gehirns aus den Signalen des Gehirnaktivitätssensors (12) und Puls-Daten aus den Signalen des kardio-vaskulären Sensors (14) in Bezug zueinander setzbar sind und die Aufgabe in Korrelation zu den ermittelten Signalen ausgesucht wird, wobei die Aufgabe aus zumindest einem Reiz ausgesucht ist, dem der Benutzer ausgesetzt wird, wobei der Reiz einen Geruch, ein Bild, eine Farbe, ein Muster, einen sensitiven Reiz, eine Melodie und/oder ein Geräusch aufweist, wobei die Vorrichtung (10) ein Cardiogerät (26) umfasst, wobei die Ausgabeeinheit (18) eine Steuerung für das Cardiogerät (26) aufweist, durch die Parameter des Cardiogerätes (26) steuerbar sind, wobei ein Leistungswiderstand des Cardiogerätes (26) so gesteuert wird, dass ein Trainingspuls erreicht wird, wobei der Trainingspuls der Puls ist, für den die Neuroplastizität im Gehirn des Benutzers optimal ist.

**2.** Vorrichtung (10) nach Anspruch 1, **dadurch gekennzeichnet, dass** die Vorrichtung (10) zumindest ein Eingabegerät (24) umfasst.

**Claims**

**1.** Device (10) for neuro-vascular stimulation, at least comprising at least one brain activity sensor (12), from the signals of which neuronal data of the brain can be determined, the brain activity sensor (12) comprising EEG electrodes and a sensor for measuring a blood flow of the brain, from the signals of which blood flow data of the brain can be determined, at least one cardio-vascular sensor (14), from the signals of which pulse data of the user can be determined, at least one computing unit (16) and at least one output unit (18), wherein the computing unit (16) has at least one task algorithm (20), wherein signals from at least the brain activity sensor (12) and the cardio-vascular sensor (14) can be received by means of the computing unit (16), and a task (22) which is correlated with at least the signals from at least the brain activity sensor (12) and the signals from the cardio-vascular sensor (14), can be determined by means of the task algorithm (20) and can be output to the user of the device (10) by means of the output unit (18), wherein at least neuronal data of the brain from the signals of the brain activity sensor (12) and pulse data from the signals of the cardio-vascular sensor (14) can be set in relation to one another by means of the task algorithm (20) and the task is selected in correlation with the determined signals, wherein the task is selected from at least one stimulus to which the user is exposed, wherein the stimulus comprises a smell, an image, a color, a pattern, a sensitive stimulus, a melody and/or a sound, wherein the device (10) comprises a cardio device (26), wherein the output unit (18) comprises a controller for the cardio device (26), by which parameters of the cardio device (26) are controllable, wherein a power resistance of the cardio device (26) is controlled so that a training pulse is achieved, wherein the training pulse is the pulse for which the neuroplasticity in the brain of the user is optimal .

**2.** The device (10) according to claim 1, **characterized in that** the device (10) comprises at least one input device (24).

**Revendications**

**1.** Dispositif (10) pour la stimulation neuro-vasculaire, comprenant au moins un capteur d'activité cérébrale (12) dont les signaux permettent de déterminer des données neuronales du cerveau, le capteur d'activité cérébrale (12) comprenant des électrodes EEG et un capteur pour mesurer une circulation sanguine du cerveau, dont les signaux permettent de déterminer des données de circulation sanguine du cerveau, au moins un capteur cardio-vasculaire (14) dont les signaux permettent de déterminer des données de pouls de l'utilisateur, au moins une unité de calcul (16) et au moins une unité de sortie (18), dans lequel

l'unité de calcul (16) présente au moins un algorithme de tâche (20),
l'unité de calcul (16) permet de recevoir des signaux d'au moins le capteur d'activité cérébrale (12) et du capteur cardio-vasculaire (14), et l'algorithme de tâche (20) permet de déterminer une tâche (22) qui est en corrélation avec au moins les signaux d'au moins le capteur d'activité cérébrale (12) et avec les signaux du capteur cardio-vasculaire (14), et l'unité de sortie (18) permet de fournir ladite tâche à l'utilisateur du dispositif (10),
l'algorithme de tâche (20) permet de mettre en relation au moins des données neuronales du cerveau provenant des signaux du capteur d'activité cérébrale (12) et des données de pouls provenant des signaux du capteur cardio-vasculaire (14), et de choisir la tâche en corrélation avec les signaux déterminés,
la tâche est choisie à partir d'au moins un stimulus auquel l'utilisateur est exposé,
le stimulus inclut une odeur, une image, une couleur, un motif, un stimulus sensible, une mélodie et/ou un bruit,
le dispositif (10) comprend un appareil d'entraînement cardio-vasculaire (26),
l'unité de sortie (18) présente une commande pour l'appareil d'entraînement cardio-vasculaire (26), laquelle permet de commander des paramètres de l'appareil d'entraînement cardio-vasculaire (26),
une résistance de puissance de l'appareil d'entraînement cardio-vasculaire (26) est commandée de telle sorte qu'un pouls d'entraînement soit atteint, le pouls d'entraînement étant le pouls pour lequel la neuroplasticité dans le cerveau de l'utilisateur est optimale.

**2.** Dispositif (10) selon la revendication 1,
**caractérisé en ce que** le dispositif (10) comprend au moins un appareil d'entrée (24).

Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20010066005 A1
- WO 2015027079 A1
- US 2015066104 A1
- EP 2388680 A1

**In der Beschreibung aufgeführte Nicht-Patentliteratur**

- **KRISTINA SCHAAFF**. Enhancing mobile working memory training by using affective feedback. *International Conference on Mobile Learning*, 01 March 2013, ISBN 978-972-893-981-6, 269-273
- **TAKAHASHI M. et al.** Experimental study toward mutual adaptive interface. *Robot and Human Communication*, 1994
- RO-MAN. IEEE, 18 July 1994, 271-276